# EUROPEAN PATENT SPECIFICATION

(11) **EP 0 509 553 B1**
(45) Date of publication and mention of the grant of the patent: **09.08.2000**
(21) Application number: 92110196.0
(22) Date of filing: 30.05.1985
(51) Int. Cl.: C07K 16/00, C07K 16/28

(54) **Anti-lymphotoxin antibody, its production and use**
Anti-Lymphotoxin-Antikörper, Herstellung und Verwendung
Anticorps anti-lymphotoxine, production et utilisation

(30) Priority: 31.05.1984 US 616503; 31.05.1984 US 616502; 09.05.1985 US 732312
(43) Date of publication of application: 21.10.1992
(62) Divisional of application: 85303818.0
(73) Proprietor: GENENTECH, INC., South San Francisco California 94080 (US)
(72) Inventor: Aggarwal, Bharat Bhushan, San Mateo, California 94403 (US); Gray, Patrick William, Seattle, WA 98112 (US); Bringman, Timothy Scott, Oakland, California 94611 (US); Nedwin, Glenn Evan, Davis California 95616 (US)
(74) Representative: Armitage, Ian Michael

(56) References cited:
- NATURE. vol. 312, no. 5596, January 1985, LONDON GB pages 721 - 724; P.W.GRAY ET AL.: 'Cloning and expression of cDNA for human lymphotoxin, a lymphokine with tumour necrosis activity'

## Description

This application relates to antibodies to lymphotoxin and derivatives thereof. It is divided out of EP 164965 (85303818.0), herein referred to as the patent specification.

Lymphotoxin was first identified as a biological factor with anticellular activity on neoplastic cell lines. An activity identified as lymphotoxin and obtained from mitogen-stimulated lymphocytes is associated with a spectrum of cytotoxic activities ranging from cytostasis of certain tumor cell lines to marked cytolysis of other transformed cells. However, lymphotoxin activity is characterized by little or no anticellular activity on primary cell cultures and normal cell lines tested. This putative discriminating anticellular property of lymphotoxin led to in vivo studies which suggest that lymphotoxin nay have a potent antitumor activity.

Lymphotoxin is the term applied to what has been described as a family of molecules. Lymphotoxin molecules have been identified as glycoproteins divided into five molecular weight classes, each of which in turn is heterogenous with respect to charge. The human alpha (MW 70-90,000) and beta (MW 25-50,000) classes appear to predominate in most lymphocyte supernatants. The alpha MW classes can be separated by charge into at least seven subclasses, while the beta subclass has been separated into two distinct subclasses (G. Granger et al. in Mozes et al., Ed., 1981, Cellular Responses to Molecular Modulators pp 287-310). Also identified have been complex (MW >200,000) and gamma (MW 10-20,000) lymphotoxin forms. The various lymphotoxin forms and classes differ from one another in their stability and kinetics of appearance in culture. Furthermore, they may aggregate together with the complex class under conditions of low ionic strength. The lower molecular weight classes of lymphotoxins have been disclosed to be relatively unstable and weakly cell lytic compared to the higher molecular weight classes (Hiserodt et al., 1976, "Cell. Immun." 26: 211; Granger et al. in De Weck et al. Ed., 1980 Biochemical Characterization of Lymphokines pp 279-283). Gamma class activity has not been studied extensively because of its instability (G. Granger et al., 1978 "Cellular Immunology" 38: 388-402). The beta class also has been reported to be unstable (Walker et al., "J. of Immun." 116[3]: 807-815 [March 1976]).

It should be understood that lymphokine terminology is not uniform. At present, the names given to cell culture products are largely a function of the cells which are believed to elaborate the product and the performance of the products in biological assays. However, these products remain poorly characterized in large measure because many studies have been conducted with partially pure preparations and because the assays used to characterize the products are not molecule-specific and in any case are subject to considerable variation. The true identity of the various cytotoxic factors will remain unknown in the absence of standard terminology based on clearly assayable distinguishing characteristics such as amino acid sequences or immune epitopes. As examples of other names given to cytotoxic cell culture products are tumor necrosis factor, NK cell cytotoxic factor, hemorrhagic necrosis factor and macrophage cytotoxin or cytotoxic factor.

Rabbit antisera has been described in the literature which is capable of neutralizing the cytolytic activity of various cytotoxins, including substances identified as lymphotoxin (Yamamoto et al."Cell. Immun." 38: 403-416 (1978); Gately et al., "Cell. Immun." 27: 82-93 (1976); Hiserodt et al., "J. of Immun." 119(2): 374-380 (1977); Zacharchuk et al., "P.N.A.S. USA" 80: 6341-6345 (October 1983); Ruddle et al., "Lymphokine Research" 2(1) 23-31 (1983); Mannel et al., "Infection and Immunity" 33(1): 156-164 (1981); Wallach et al. E. De Maeyer et al. Ed. The Biology of the Interferon System pp 293-302 (Pub. September 1983) and Stone-Wolff et al., "J. Exp. Med." 159: 828-843 (March 1984). Since this antisera is polyclonal it contains a multiplicity of antibodies directed against the immunogen lymphotoxin. Any one or more of these antibodies is acting to neutralize the "lymphotoxin" activity. Further, the literature reports generally are unclear as to the molecular identity of the substance responsible for lymphotoxin activity that was used as the immunogen. What is needed for diagnosis and immunoaffinity purification procedures is a monospecific antibody directed against a clearly and unambiguously identified lymphotoxin molecule. It is an objective of this invention to provide such an antibody.

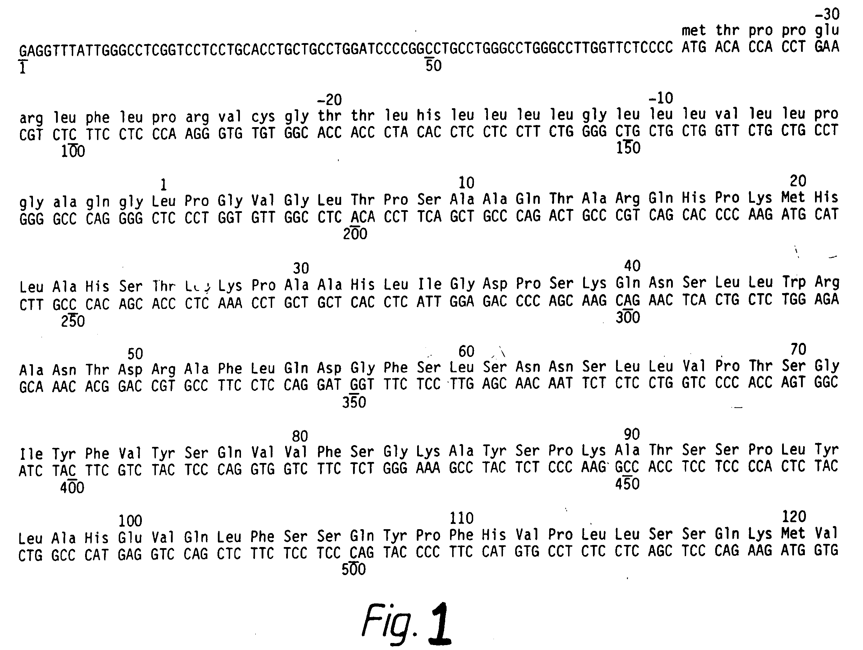

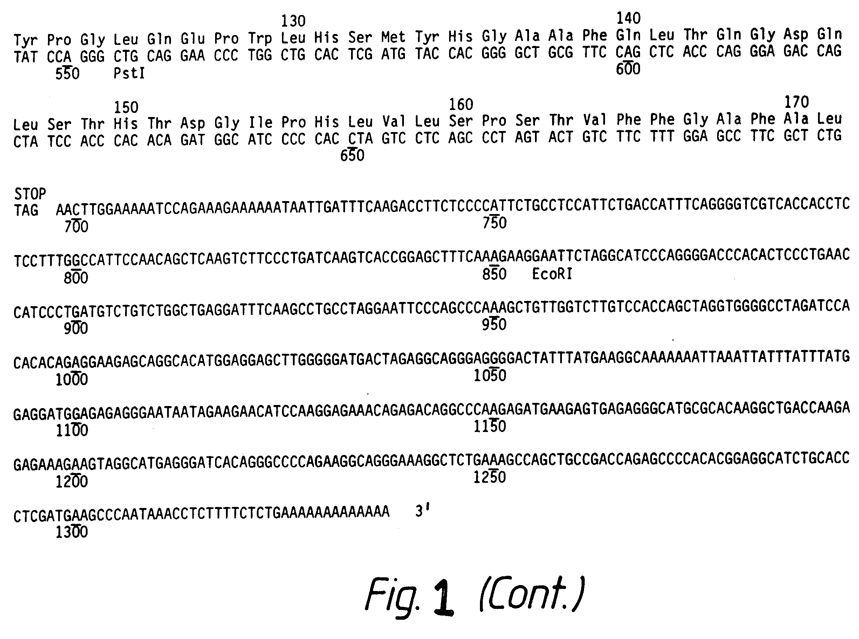

The parent specification EP 164965A discloses the production by recombinant DNA technology of a particular lymphotoxin and variants and homologs thereof. Fig. 1 of the present application (corresponding to Fig. 2a of the parent) shows the complete amino acid sequence for prelymphotoxin, its coding DNA plus 5' and 3' flanking untranslated regions.

Such lymphotoxin can be purified from culture supernatants or lysates by immunoaffinity adsorption using insolubilized lymphotoxin-neutralizing antibody. This antibody, which is most efficiently produced in monoclonal cell culture, is raised in mice by immunization with alum-adsorbed lymphotoxin. Thus, the present invention provides inter alia antibody suitable for that purpose.

### Detailed description

Lymphotoxin is defined for the purposes of this application as a biologically active polypeptide having a region demonstrating substantial structural amino acid homology with at least a portion of the lymphotoxin amino acid sequence shown in Fig. 1. Biological activity is defined as preferential, cytotoxic activity as defined below, immunological cross-reactivity with a cytotoxic lymphotoxin or the ability to compete with cytotoxic lymphotoxin for lymphotoxin cell surface receptors. In the latter two instances the lymphotoxin need not be cytotoxic per se. Immunologically cross-reactive mutants are useful as immunogens for raising anti-lymphotoxin in animals, e.g. for the preparation of immunoassay reagents, while non-cytotoxic competitive mutants find utility as labelled reagents in competitive-type immunoassays for biologically active lymphotoxin.

Preferential cytotoxic activity is defined as the preferential destruction or growth inhibition of tumor cells in vivo or in vitro when compared to normal cells under the same conditions. Destruction of tumor cells by lysis in vitro or necrosis in vivo is the preferred assay endpoint, although cytostasis or antiproliferative activity also is used satisfactorily.

Suitable assays for detecting the anticellular activities of lymphotoxin are described in B. Aggarwal, et al., 1984, "J. Biol. Chem." 259 (1), 686-691 and E. Carswell, et al., 1975, "Proc. Natl. Acad. Sci. USA" 72, 3666-3670.

Lymphotoxin specific activity is defined herein in terms of target cell lysis, rather than cytostasis. One unit of lymphotoxin is defined as the amount required for 50 percent lysis of target cells plated in each well as is further described in Example 1 of EP 164965. However, other methods for determining cytotoxic activity are acceptable.

Substantial structural homology means that greater than 60 percent, and usually greater than 70 percent of the amino acid residues in the polypeptide are the same or conservative substitutions for the corresponding residue(s) in the sequence of Fig. 1.

Not all of the sequence of a lymphotoxin polypeptide need be homologous with the Fig. 1 sequence. Only a portion thereof need be homologous with any portion of the Fig. 1 sequence so long as the candidate exhibits the required biological activity. Generally, homology should be demonstrable for regions of about from 20 to 100 amino acid residues, recognizing that occasional gaps may need to be introduced in order to maximize the homology.

Less homology is required for polypeptides to fall within the definition if the region of homology with the Fig. 1 sequence is not in one of the lymphotoxin key regions, i.e. regions that are important for cytotoxic activity. The key regions of the Fig. 1 sequence are believed to be about residues 162-171, 52-83 and 127-148.

Lymphotoxin is defined to specifically exclude human tumor necrosis factor or its natural animal analogues (D. Pennica et al., "Nature" 312:20/27 December, 1984, pp. 724-729 and B. Aggarwal et al., "J. Biol. Chem." 260[4]: 2345-2354 [1985]).

Structurally similar refers to dominant characteristics of the amino acid side chains such as basic, neutral or acid, hydrophilic or hydrophobic, or the presence or absence of steric bulk. Substitution of one structurally similar amino acid for another generally is known in the art as a conservative substitution.

A significant factor in establishing the identity of a polypeptide as lymphotoxin is the ability of antisera which are capable of substantially neutralizing the cytolytic activity of substantially homogeneous, lymphoblastoid (or natural) lymphotoxin to also substantially neutralize the cytolytic activity of the polypeptide in question. However it will be recognized that immunological identity and cytotoxic identity are not necessarily coextensive. A neutralizing antibody for the lymphotoxin of Fig. 1 may not bind a candidate protein because the neutralizing antibody happens to be directed to a site on lymphotoxin that merely neighbors a region that is critical to lymphotoxin cytotoxic activity, but which acts as a neutralizing antibody by steric hinderance of the lymphotoxin active site. A candidate protein mutated in this innocuous region might no longer bind the neutralizing antibody, but it would nonetheless be lymphotoxin in terms of substantial homology and biological activity.

Lymphotoxin obtained by culture of lymphoblastoid cell lines has been determined to have the following characteristics: A molecular weight of 20,000 or 25,000, depending upon the degree of glycosylation and N-terminal heterogeneity; glycosylation at Asn+62 (Fig. 1 ); a tendency to aggregate, particularly to organize into multimers; an isoelectric point of about 5.8; pH lability (a loss of >50 percent of cytolytic activity when stored for 24 hours in ammonium bicarbonate buffer at 10 µg/ml concentration with pH levels less than about 5 or greater than about 10); and substantial losses in activity upon incubation in aqueous solution for 5 min. at 80°C. Two lymphoblastoid lymphotoxin molecular weight species have been identified. The 25,000 da species of lymphoblastoid lymphotoxin has an amino-terminal leucine residue. Polypeptides having the primary amino acid sequence of the 25,000 da species are called leucyl amino-terminal lymphotoxin. The 20,000 da species of lymphoblastoid lymphotoxin is characterized by an amino-terminal histidine and corresponding sequences are termed histidyl amino-terminal lymphotoxin. It is important to observe that these characteristics describe the native or wild type human lymphotoxin obtained from lymphoblastoid cell cultures. While lymphotoxin as defined herein includes native, glycosylated lymphotoxin, other related cytotoxic polypeptides many fall within the scope of the definition. For example, the glycosylation ordinarily associated with an animal lymphotoxin may be modified upon expression in a heterologous recombinant eukaryotic host cell, thereby bringing the modified lymphotoxin outside of the molecular weights or isoelectric point established for human lymphoblastoid lymphotoxin. Lymphotoxin which is entirely unglycosylated is produced in recombinant bacterial culture with its molecular weight, isoelectric point and other characteristics correspondingly modified. In addition, post-translational processing of pre lymphotoxin from a first animal species in a cell line derived from another animal species may result in a different amino-terminal residue than is ordinarily the case for the first animal species. Similarly, the mutagenesis procedures provided herein, for example, will enable one to vary the amino acid sequence and N-terminus of lymphotoxin, thereby modifying the pH stability, isoelectric point and the like.

The translated amino acid sequence for human lymphotoxin is described in Fig. 1 . Note that this sequence includes a 34 residue presequence which is believed to be removed during normal processing of the translated transcript in human cells (herein, together with its mutants, "pre lymphotoxin"), resulting in the leucyl amino terminal species. The histidyl amino-terminal species is homologous to the leucyl amino-terminal species except that the first 23 amino acids of the leucyl amino-terminal species are absent. All three species, i.e. pre lymphotoxin, leucyl amino-terminal lymphotoxin and histidyl amino-terminal lymphotoxin, as well as their methionyl, modified methionyl, mutant and unglycosylated forms, are included within the scope of lymphotoxin. The unglycosylated leucyl and histidyl amino-terminal species will have lower molecular weights than described above for the homologous species from lymphoblastoid cells.

Pre lymphotoxin is a species of lymphotoxin included within the foregoing definition. It is characterized by the presence of a signal (or leader) polypeptide at the amino terminus of the molecule. Generally, the native signal polypeptide of lymphotoxin is proteolytically cleaved from lymphotoxin as part of the secretory process in which the protein is secreted from the cell. The signal peptide may be microbial or mammalian (including the native, 34 residue presequence), but it preferably is a signal which is homologous to the host cell. Some signal-lymphotoxin fusions are not recognized or "processed" by the host cell into N-terminal met-free lymphotoxin. Such fusions containing microbial signals have utility for example as lymphotoxin immunogens.

Note that the language "capable" of cytotoxic activity means that lymphotoxin includes polypeptides which can be converted, as by enzymatic hydrolysis, from an inactive state analogous to a zymogen to a polypeptide fragment which exhibits the desired biological activity. The language "capable" of in vitro or in vivo cytotoxic activity is intended to embrace noncytotoxic polypeptides which can be converted, as by enzymatic hydrolysis, from an inactive state analogous to a zymogen to a polypeptide fragment which exhibits the definitional biological activity. Typically, inactive precursors will be fusion proteins in which lymphotoxin is linked by a peptide bond at its carboxyl terminus to another protein or polypeptide. The sequence at this peptide bond or nearby is selected, so as to be susceptible to proteolytic hydrolysis to release lymphotoxin, either in vivo or, as part of a manufacturing protocol, in vitro. Typical linking sequences are lys-lys or arg-lys. The nonlymphotoxin component to such a prolymphotoxin is preferably a homologous protein so as to minimize the immunogenicity of the fusion. The homologous protein should be innocuous and not bind to cell surfaces. The lymphotoxin that is so generated then will exhibit the definitionally-required cytotoxic activity.

While lymphotoxin ordinarily means human lymphotoxin, lymphotoxin from sources such as murine, porcine, equine or bovine is included within the definition of lymphotoxin so long as it otherwise meets the standards described above for homologous regions and biological activity. For example, bovine and murine lymphotoxins have been found to be highly (about 80 percent) homologous with human lymphotoxin. Lymphotoxin is not species specific, e.g., human lymphotoxin is active on mouse tumors and neoplastic cell lines. Therefore, lymphotoxin from one species can be used in therapy of another.

Lymphotoxin also includes multimeric forms. Lymphotoxin spontaneously aggregates into multimers, usually dimers or higher multimers. Multimers are cytotoxic and accordingly are suitable for use in in vivo therapy. Lymphotoxin is expressed in recombinant hosts as a monomer. However, lymphotoxin thereafter tends to spontaneously form multimers. Homogeneous multimers or a mixture of different multimers are therapeutically useful.

Variant lymphotoxins include predetermined or targeted, i.e. site specific, mutations of the Fig. 1 molecule or its fragments. Variant lymphotoxins are defined as polypeptides otherwise meeting the defined characteristics of lymphotoxin but which are characterized by an amino acid sequence that differs from that of Fig. 1 , whether by omission, substitution or insertion of residues. The nonhuman lymphotoxins described herein, and alleles of human lymphotoxin, are to be considered variant lymphotoxins, as are site-directed mutants having no natural counterpart. The objective of mutagenesis is to construct DNA that encodes lymphotoxin as defined above but exhibits characteristics that modify the biological activity of natural lymphotoxin or facilitate the manufacture of lymphotoxin. For example, the lysine +89 codon is mutated in order to express a histidine residue in place of the lysine residue. The histidine +89 is no longer hydrolyzed by trypsin (which generally cleaves proteins at an arg-X or lys-X bond). Protease resistance is expected to confer greater biological half life on the mutant than is the case for lymphotoxin having the sequence of Fig. 1 (or a fragment thereof). Other lymphotoxin lysine or arginine residues may be mutated to histidine, for example lysine +28, lysine +19 or arginine +15.

As discussed above, certain regions of the lymphotoxin molecule exhibit substantial homology with a similarly-active protein designated tumor necrosis factor. Amino acid residues in and immediately flanking these substantially homologous regions are preferred for mutagenesis directed to identifying lymphotoxin nutants that exhibit variant biological or cytotoxic activity. Such mutants are made by methods known per se and then screened for the desired biological activity, e.g. increased cytotoxicity towards the particular neoplasm being treated or, in the case of lymphotoxin species intended for immunization of animals, the ability to elicit a more potent immune response. Examples of such lymphotoxin variants are as follows: Ala+168 is mutated to a branched chain amino acid (val, ile, or leu); a hydrophobic amino acid (e.g., phe, val, ile or leu) is inserted between thr+163 and val+164; tyrosine substituted for thr+163; lysine substituted for ser+82; isoleucine, leucine, phenylalanine, valine or histidine substituted for ser+42; glutamine, tryptophan, serine or histidine substituted for lys+84; ser+82 deleted; a hydrophobic di-or tripeptide fused to leu+171; aspartic acid or lysine substituted for thr+163; ala-lys inserted between glu+127 and pro+128; lysine or glycine substituted for ser+70; tyrosine substituted for thr+69; arginine or histidine substituted for lys+28; arginine or lysine substituted for his+32; proline, serine, threonine, tyrosine or glutamic acid substituted for asp+36; tyrosine, methionine or glutamic acid substituted for ser+38; threonine, tyrosine, histidine, or lysine substituted for ser+61; aspartic acid, serine or tyrosine substituted for gly+124; arginine, lysine, tyrosine, tryptophan or proline substituted for his+135; aspartic acid substituted for thr+142; and lysine or threonine substituted for gln+146.

A particularly desirable group of mutants are those in which the methionine residues at human lymphotoxin residues +20, +120 and +133 are deleted or, preferably, substituted for by the corresponding residues found in the lymphotoxins of other species such as are described elsewhere herein. For example met+20, +120 and +133 are substituted by threonine, serine and valine, respectively. These are the corresponding residues in bovine lymphotoxin. The substitution is effected in the manner described in Example 9 of EP 164965A except that met⁺133 is mutated to val by a further step of mutagenesis using M13 Mp8 phage in accord with methods known per se. This mutant animal species-hybrid lymphotoxin DNA is used in place of the leucyl amino-terminal DNA of Example 7 of the parent specification and expressed as a fusion. Following known procedures, cyanogen bromide is used to cleave the STII signal from the hybrid lymphotoxin and the mature leucyl amino-terminal lymphotoxin variant recovered.

Other useful variant lymphotoxins are those in which residues from tumor necrosis factor are substituted for corresponding lymphotoxin residues to produce hybrid tumor necrosis factor-lymphotoxin variants. A representative example is the substitution of the first 8, 9 or 10 residues of mature tumor necrosis factor (e.g., val-arg-ser-ser-ser-arg-thr-pro-ser-asp-) for the first 27 residues of leucyl amino-terminal lymphotoxin. This variant is more likely to be N-terminal demethionylated upon direct expression in E. coli.

While the mutation site is predetermined, it is unnecessary that the mutation per se be predetermined. For example, in order to optimize the performance of the mutant histidine +89 lymphotoxin, random mutagenesis is conducted at the codon for lysine +89 and the expressed lymphotoxin mutants screened for the optimal combination of cytotoxic activity and protease resistance.

Lymphotoxin also may contain insertions, usually on the order of about from 1 to 10 amino acid residues, or deletions of about from 1 to 30 residues. Substitutions, deletions, insertions or any subcombination may be combined to arrive at a final construct. Insertions include amino or carboxyl-terminal fusions, e.g. a hydrophobic extension added to the carboxyl terminus. Preferably, however, only substitution mutagenesis is conducted. Obviously, the mutations in the encoding DNA must not place the sequence out of reading frame and preferably will not create complementary regions that could produce secondary mRNA structure. Extracts of E. coli transformed with vectors containing DNA encoding lymphotoxin mutants having a deletion of the last 16 carboxy terminal amino acids or deletion of the first about 33 amino terminal residues of leucyl amino-terminal lymphotoxin exhibited no cytotoxic activity. However, the reasons for lack of activity are not known and could have been any of those set forth in Example 1 of the parent EP 164965A.

Not all mutations in the DNA which encodes the lymphotoxin will be expressed in the ultimated product of recombinant cell culture. For example, a major class of DNA substitution mutations are those DNAs in which a different secretory leader has been substituted for the Fig. 1 secretory leader, either by deletions within the 34 residue leader or by substitutions, which exchange of most or all of the native leader for a leader more likely to be recognized by the intended host. For example, in constructing a procaryotic expression vector the Fig. 1 secretory leader is deleted in favor of the bacterial alkaline phosphatase or heat stable enterotoxin II leaders, and for yeast the Fig. 1 leader is substituted in favor of the yeast invertase, alpha factor or acid phosphatase leaders. This is not to imply, however, that the human secretory leader is not recognized by hosts other than human cell lines. When the secretory leader is "recognized" by the host, the fusion protein consisting of lymphotoxin and the leader ordinarily is cleaved at the leader-lymphotoxin peptide bond in the same event that leads to secretion of the lymphotoxin. Thus, even though a mutant DNA is used to transform the host the resulting product lymphotoxin may be either a fused or native lymphotoxin, depending upon the efficacy of the host cell in processing the fusion.

Another major class of DNA mutants that are not expressed as lymphotoxin variants are nucleotide substitutions made to enhance expression, primarily by avoiding stem-loop structures in the transcribed mRNA (see copending U.S.S.N. 303,687, EP 75444) or to provide codons that are more readily transcribed by the selected host, e.g. the well-known E. coli preference codons for E. coli expression.

The mutant nucleic acid is made by known methods per se (A. Hui et al., 1984, "The EMBO Journal" 3(3): 623-629; J. Adelman et al., 1983, "DNA" 2(3): 183-193; U.K. Patent Application 2,130,219A; G. Winter et al., 1902, "Nature" 299: 756-758; and R. Wallace et al., 1981, "Nucleic Acids Research" 9(15): 3647-3656). These methods include M13 phage mutagenesis, synthesis of the mutant lymphotoxin gene as described in Example 1 et seq. of EP 164965A as are or will become known in the art.

A method is provided herein for obtaining lymphotoxin-neutralizing antibody. Neutralizing antibody is defined as antibody that is capable of immunologically binding lymphotoxin as defined herein in such a way as to substantially reduce its activity in cytostatic or cytolytic lymphotoxin activity assays such as the murine L929 assay described below. The fact that the antibody is capable of neutralizing lymphotoxin activity does not mean that the antibody must bind directly to the lymphotoxin active or receptor binding site. The antibody may still substantially neutralize lymphotoxin activity if it sterically binds to a region which adjacent to the critical site, i.e., adjacent in the sense of conformationally adjacent and not necessarily adjacent from the point of view of amino acid sequence.

In attempting to prepare a neutralizing monoclonal antibody against lymphotoxin, it proved difficult to immunize mice in a fashion such that lymphotoxin neutralizing antibody is generated or raised in the animals. Neither immunization with lymphoblastoid lymphotoxin nor glutaraldehyde cross-linked lymphotoxin resulted in any detectable neutralizing antibody in the serum of immunized mice, even though the mice did raise non-neutralizing anti-lymphotoxin antibody detectable by enzyme immunoassay. However, immunization with a lymphotoxin-alum (aluminum hydroxide or alumina, Al₂O₃.3H₂O) adsorption complex will raise neutralizing antibody even in animals which had failed to generate the activity prior to immunization with the alum complex. Preparation of alum and its use in the production of antiserum are disclosed in C. Williams, et al.,eds., 1967, Methods in Immunology and Immunochemistry I, pp 197-229.

Fusions of spleen cells from animals producing neutralizing antibody with murine myeloma cells are made. On the average, about 50 to 100 clones will have to be screened to identify one which synthesizes neutralizing antibody. The process for screening the clones for the desired activity is routine and well within the skill of the ordinary artisan, and can be reproduced with minimal experimental effort.

The serum, plasma or IgG fractions from the immunized animal, as well as immunoglobulins secreted by hybridomas generated from the spleen or lymph cell of immunized animals, are all satisfactory for use herein. In a preferred embodiment the neutralizing antibody is obtained essentially free of other anti-lymphotoxin antibody in hybridoma culture.

The neutralizing antibody is immobilized by adsorption to surfaces, e.g., thermoplastics such as polystyrene, or covalently bound to matrices such as cyanogen bromide-activated Sepharose. It then is used in immunoassays or in immunoaffinity purification. Since the antibody is a neutralizing antibody it is most likely only to adsorb or detect biologically active lymphotoxin or fragments thereof. The antibody is particularly useful in immunoradiometric ("sandwich") immunoassays in concert with a non-neutralizing anti-lymphotoxin monoclonal antibody or a polyclonal antiserum which contains non-neutralizing anti-lymphotoxin. The immunoassay is conducted using either the neutralizing or non-neutralizing antibody as the labelled component, which labelling is effective with a detectable substance such as a fluorescent, chemiluminescent or radioisotopic label in accord with methods known in the art. For competitive-type lymphotoxin immunoassays, lymphotoxin is labelled in the same fashion. Chloramine-T radioiodination is suitable for both lymphotoxin and lymphotoxin antibody tracer preparation, or the method described in J. Klostergaard et al., "Mol. Immun." 18: 455 (1980) is used.

### EXAMPLE 1

### Method for Making Monoclonal Murine Antibody Capable of Neutralizing Lymphotoxin

Purified lymphoblastoid lymphotoxin obtained in Example 1 of the parent specification EP 164965A was dialyzed against phosphate buffered saline (PBS). 200 µg of lymphotoxin/ml were contained in the dialysate. Glutaraldehyde was added to the dialysate to a concentration of 70mM glutaraldehyde, the mixture incubated for 2 hours at room temperature, more glutaraldehyde added to bring its total added concentration up to 140 mM, incubation continued for another 6 hours and the mixture then dialyzed against PBS. 50 µg of the glutaraldehyde cross-linked lymphotoxin (hereafter, "polylymphotoxin") and 0.5 ml of Freund's complete adjuvant was injected subcutaneously into mice (strain BALB/c). After one week, the mouse was booster immunized with 50 µg of polylymphotoxin and 0.5 ml of Freund's incomplete adjuvant, half intramuscularly and half into the peritoneal cavity. Serum was harvested after 7 days and assayed for anti-lymphotoxin activity by an ELISA assay.

The ELISA assay was conducted as follows: A buffered solution of purified lymphotoxin was placed in microtiter wells and permitted to coat the wells with about 100 ng of lymphotoxin each. The unadsorbed lymphotoxin solution was aspirated from the wells. 50 µl of appropriately diluted test sample was combined with 100 µl PBS containing 5 mg/ml bovine serum albumin (PBS-BSA buffer) and added to each well, incubated for 2 hours at room temperature, washed with PBS containing 0.05 percent Tween 20, 100 µl of horse radish peroxidase-labelled goat anti-mouse IgG in PBS-BSA buffer added to each well and incubated for 1 hour. Each well was washed with PBS containing 0.05 percent Tween 20 and then citrate phosphate buffer, pH5, containing 0:1 mg o-phenylene diamine/ml (substrate solution) and aqueous 30 percent H₂O₂ (at a proportion of 4 µl of 30 percent v/v H₂O₂ per 10 ml of substrate solution) was added to each well. The wells were incubated for 30 min., the reaction stopped with 50 µl 2.5M sulfuric acid and adsorbance measured at 492 nm. Wells which showed adsorbance greater than 1 O.D. were considered anti-lymphotoxin positive.

Test samples also were assayed for the ability to neutralize the cytolytic activity of lymphotoxin in the murine L929 assay. Serum harvested from immunized animals or hybridoma supernatants were diluted as required into RPMI-1640 medium containing 10 percent fetal bovine serum and about 100 lymphotoxin units/ml and plated into microtiter wells containing cultured L929 cells as is otherwise conventional in the cytolysis assay. In the control, all cells were lysed. Neutralizing antibody was detected by failure of the lymphotoxin to lyse L929 cells.

The animal immunized with glutaraldehyde-polymerized lymphotoxin raised antibodies which were active in the ELISA assay, but no serum neutralizing activity was detected.

A suspension containing 100 µg lymphotoxin and 1 ml of a 1.64 percent w/v suspension of aluminum hydroxide [(Al(OH)₃] was prepared and used to immunize the same mouse. The mouse was injected with 100 µl of the suspension intramuscularly and 400 µl intraperitoneally. After one week the mouse was injected intravenously with 10 µg of unpolymerized and unadsorbed lymphoblastoid lymphotoxin in 100 µl of PBS. A test of a 1/80 dilution of the animal's serum three days later indicated the presence lymphotoxin neutralizing antibody.

The spleen from this animal was harvested. 3x10⁷ spleen cells were fused with 5x10⁷ murine myeloma cells and plated into microtiter wells containing HAT medium and about 3000 peritoneal macrophages/microtiter well according to the procedure of S. Fazekas De St. Groth, 1980, "J. Immunol. Meth." 35: 1-21. Hybridomas from wells containing supernatants which were positive in the above ELISA assay were grown in 1 ml volume of DMEM medium with 20 percent fetal calf serum, 10 percent NCTC-135 medium, 5 x 10⁻⁵ M betamercaptoethanol and HAT, distributed into microtiter wells at a statistical average of one cell per well and then cultured in a 1 or 5 ml volume of the same medium. Supernatants were thereafter assayed for neutralizing antibody. Statistically, about 2 percent of the ELISA positive hybridomas from the aluminum hydroxide immunization synthesized neutralizing antibody. High affinity lymphotoxin antibody optionally is selected from this group of hybridomas.

### EXAMPLE 2

### Immunoaffinity Purification of Lymphotoxin

A murine monoclonal cell line secreting anti-lymphotoxin (Example 1) was grown in mice and purified from ascites fluid by ion exchange chromatography. The anion exchange eluate was coupled to cyanogen bromide activated Sepharose at a concentration of 2 mg/ml resin. A 20 ml column was equilibrated consecutively with TBS (containing 0.05 M Tris-HCl, pH 7.0, 0.15 M sodium chloride, and 2mM EDTA); then with elution buffer (containing 0.1 M acetic acid, pH 4.5, 150 mM sodium chloride); and finally with TBS. A 40 percent saturated ammonium sulfate precipitate of pLTtrp1-transformed E. coli sonicated lysate (previously clarified by centrifugation) was suspended in 0.1 M Tris-HCl, pH 7.4, and 5 mM EDTA and loaded onto the column at a rate of one column volume per hour. Following extensive washing with TBS containing 0.05 percent Tween-20, specifically bound material was eluted with the elution buffer, the pH immediately adjusted to 7.8 with 0.1 volume 1 M Tris-HCl, pH 8.5, and stored at 4°C. The specific activity of this purified lymphotoxin was 2-10x10⁷ units/mg, as measured in the above murine L-929 assay.

The eluate contained most of the activity loaded onto the column. The majority of the total eluate protein migrated as a single band under both reducing and nonreducing conditions in SDS-polyacrylamide gel electrophoresis. The mobility of this band corresponds to approximately 18,000 MW, which is consistent with the predicted value of 18,664 MW fur unglycosylated leucyl-amino terminal lymphotoxin based on the deduced amino acid sequence. To further characterize its biological activities, the purified recombinant lymphotoxin was tested for cytolytic activity in vitro and antitumor activity in vivo.

## Claims (Claims for the following Contracting State(s): BE, CH, LI, DE, FR, GB, IT, LU, NL, SE)

1. An antibody which neutralises the cytolytic activity of
(i) lymphotoxin having the amino acid sequence 1-171 depicted in Fig. 1 hereof, or
(ii) a lymphotoxin which is capable of cytotoxic activity towards tumour cells in vitro or tumour cells in vivo and which has a region demonstrating greater than 60% structural amino acid homology with at least a portion of at least 20 amino acids of the lymphotoxin amino acid sequence 1-171 depicted in Fig. 1,
and which antibody is free of non-neutralising antibodies.

2. The antibody of claim 1 which is a monoclonal antibody.

3. The antibody of claim 1 or claim 2 labelled with a detectable substance.

4. The antibody of claim 3 wherein the detectable substance is a fluorescent, chemiluminescent or radioisotopic label.

5. The antibody of any one of claims 1 to 4 which is immobilised on a surface or matrix.

6. The antibody of claim 5 which is immobilised by adsorption or by a covalent bond to a surface or matrix.

7. A method for separating lymphotoxin from a composition, which comprises contacting the antibody of any one of claims 1 to 3 with the composition under conditions whereby lymphotoxin is adsorbed thereon, separating the antibody-adsorbed lymphotoxin from the composition, and separating the lymphotoxin from the antibody.

8. A non-therapeutic method for raising antibody against lymphotoxin, which comprises immunising an animal against glutaraldehyde-polymerised lymphotoxin and thereafter immunising the same animal against a lymphotoxin-alum adsorption complex, wherein the lymphotoxin has the amino acid sequence 1-171 depicted in Fig. 1 hereof, or has an amino acid sequence demonstrating greater than 60% structural homology with at least a portion of at least 20 amino acids of said sequence 1-171 and which displays preferential cytotoxic activity of, and/or immunological cross-reactivity with, and/or the ability to compete for lymphotoxin cell surface receptors with, cytotoxic lymphotoxin.

## Claims (Claims for the following Contracting State(s): AT)

1. A method for raising antibody against lymphotoxin, which comprises immunising an animal against glutaraldehyde-polymerised lymphotoxin and thereafter immunising the same animal against a lymphotoxin-alum adsorption complex, wherein the lymphotoxin has the amino acid sequence 1-171 depicted in Fig. 1 hereof, or has an amino acid sequence demonstrating greater than 60% structural homology with at least a portion of at least 20 amino acids of said sequence 1-171 and which displays preferential cytotoxic activity of, and/or immunological cross-reactivity with, and/or the ability to compete for lymphotoxin cell surface receptors with, cytotoxic lymphotoxin.

2. A method according to claim 1 wherein the antibody neutralises the cytolytic activity of
(i) lymphotoxin having the amino acid sequence 1-171 depicted in Fig. 1 hereof, or
(ii) a lymphotoxin which is capable of cytotoxic activity towards tumour cells in vitro or tumour cells in vivo and which has a region demonstrating greater than 60% structural amino acid homology with at least a portion of at least 20 amino acids of the lymphotoxin amino acid sequence 1-171 depicted in Fig. 1,
and is free of non-neutralising antibodies.

3. A method according to claim 1 or claim 2 wherein the antibody is a monoclonal antibody.

4. A method of any one of claims 1 to 3 wherein the antibody is labelled with a detectable substance.

5. A method of claim 4 wherein the detectable substance is a fluorescent, chemiluminescent or radioisotopic label.

6. A method of any one of claims 1 to 5 wherein the antibody is immobilised on a surface or matrix.

7. A method of claim 6 wherein the antibody is immobilized by adsorption or by a covalent bond to a surface or matrix.

8. A method for separating lymphotoxin from a composition, which comprises contacting an antibody as obtainable by the method of any one of claims 1 to 3 with the composition under conditions whereby lymphotoxin is adsorbed thereon, separating the antibody-adsorbed lymphotoxin from the composition, and separating the lymphotoxin from the antibody.

## Patentansprüche (Patentansprüche für folgende(n) Vertragsstaat(en): BE, CH, LI, DE, FR, GB, IT, LU, NL, SE)

1. Antikörper, der die zytolytische Aktivität
(i) von Lymphotoxin mit der in Fig. 1 gezeigten Aminosäuresequenz 1 bis 171 oder
(ii) eines Lymphotoxins, das zu zytotoxischer Aktivität gegenüber Tumorzellen in vitro oder Tumorzellen in vivo fähig ist und einen Bereich aufweist, der mehr als 60 % Aminosäure-Strukturhomologie mit zumindest einem Abschnitt von zumindest 20 Aminosäuren der in Fig. 1 dargestellten Lymphotoxin-Aminosäuresequenz 1 bis 171 besitzt,
neutralisiert, und der frei von nicht-neutralisierenden Antikörpern ist.

2. Antikörper nach Anspruch 1, der ein monoklonaler Antikörper ist.

3. Antikörper nach Anspruch 1 oder 2, der mit einer detektierbaren Substanz markiert ist.

4. Verfahren nach Anspruch 3, worin die detektierbare Substanz eine Fluoreszenz-, Chemolumineszenz- oder Radioisotopen-Markierung ist.

5. Antikörper nach einem der Ansprüche 1 bis 4, der an einer Oberfläche oder Matrix immobilisiert ist.

6. Antikörper nach Anspruch 5, der durch Adsorption oder über eine kovalente Bindung an eine Oberfläche oder Matrix immobilisiert ist.

7. Verfahren zum Abtrennen von Lymphotoxin von einer Zusammensetzung, welches das In-Kontakt-Bringen eines Antikörpers nach einem der Ansprüche 1 bis 3 mit der Zusammensetzung unter Bedingungen, durch die Lymphotoxin daran adsorbiert wird, das Abtrennen des Antikörper-adsorbierten Lymphotoxins von der Zusammensetzung, und das Abtrennen des Lymphotoxins vom Antikörper umfasst.

8. Nicht-therapeutisches Verfahren zum Züchten von Antikörpern gegen Lymphotoxin, welches das Immunisieren eines Tiers gegen Glutaraldehyd-polymerisiertes Lymphotoxin und das anschließende Immunisieren desselben Tiers gegen einen Lymphotoxin-Alaun-Adsorptionskomplex umfasst, worin das Lymphotoxin die in Fig. 1 dargestellte Aminosäuresequenz 1-171 aufweist oder über eine Aminosäuresequenz verfügt, die mehr als 60 % Strukturhomologie mit zumindest einem Abschnitt von zumindest 20 Aminosäuren der Sequenz 1-171 aufweist, und die preferentielle zytotoxische Aktivität von zytotoxischem Lymphotoxin und/oder immunologische Kreuzreaktivität damit und/oder die Fähigkeit zum Konkurrieren um Lymphotoxin-Zelloberflächenrezeptoren damit aufweist.

## Patentansprüche (Patentansprüche für folgende(n) Vertragsstaat(en): AT)

1. Verfahren zum Züchten von Antikörpern gegen Lymphotoxin, welches das Immunisieren eines Tiers gegen Glutaraldehyd-polymerisiertes Lymphotoxin und das anschließende Immunisieren desselben Tiers gegen einen Lymphotoxin-Alaun-Adsorptionskomplex umfasst, worin das Lymphotoxin die in Fig. 1 dargestellte Aminosäuresequenz 1-171 aufweist oder über eine Aminosäuresequenz verfügt, die mehr als 60 % Strukturhomologie mit zumindest einem Abschnitt von zumindest 20 Aminosäuren der Sequenz 1-171 aufweist, und die preferentielle zytotoxische Aktivität von zytotoxischem Lymphotoxin und/oder immunologische Kreuzreaktivität damit und/oder die Fähigkeit zum Konkurrieren um Lymphotoxin-Zelloberflächenrezeptoren damit aufweist.

2. Verfahren nach Anspruch 1, worin der Antikörper die zytolytische Aktivität
(i) von Lymphotoxin mit der in Fig. 1 gezeigten Aminosäuresequenz 1 bis 171 oder
(ii) eines Lymphotoxins, das zu zytotoxischer Aktivität gegenüber Tumorzellen in vitro oder Tumorzellen in vivo fähig ist und einen Bereich aufweist, der mehr als 60 % Aminosäure-Strukturhomologie mit zumindest einem Abschnitt von zumindest 20 Aminosäuren der in Fig. 1 dargestellten Lymphotoxin-Aminosäuresequenz 1 bis 171 aufweist,
neutralisiert und frei von nicht-neutralisierenden Antikörpern ist.

3. Verfahren nach Anspruch 1 oder 2, worin der Antikörper ein monoklonaler Antikörper ist.

4. Verfahren nach einem der Ansprüche 1 bis 3,worin der Antikörper mit einer detektierbaren Substanz markiert ist.

5. Verfahren nach Anspruch 4, worin die detektierbare Substanz eine Fluoreszenz-, Chemolumineszenz- oder Radioisotopen-Markierung ist.

6. Verfahren nach einem der Ansprüche 1 bis 5, worin der Antikörper an einer Oberfläche oder Matrix immobilisiert ist.

7. Verfahren nach Anspruch 6, worin der Antikörper durch Adsorption oder über eine kovalente Bindung an eine Oberfläche oder Matrix immobilisiert ist.

8. Verfahren zum Abtrennen von Lymphotoxin von einer Zusammensetzung, welches das In-Kontakt-Bringen eines nach dem Verfahren nach einem der Ansprüche 1 bis 3 erhältlichen Antikörpers mit der Zusammensetzung unter Bedingungen, durch die Lymphotoxin daran adsorbiert wird, das Abtrennen des Antikörper-adsorbierten Lymphotoxins von der Zusammensetzung, und das Abtrennen des Lymphotoxins vom Antikörper umfasst.

## Revendications (Revendications pour l'(les) Etat(s) contractant(s) suivant(s): BE, CH, LI, DE, FR, GB, IT, LU, NL, SE)

1. Anticorps qui neutralise l'activité cytolytique
(j) d'une lymphotoxine ayant la séquence d'aminoacides 1-171 représentée sur la figure 1, ou
(ii) d'une lymphotoxine qui est apte à présenter une activité cytotoxique sur des cellules tumorales in vitro ou des cellules tumorales in vivo et qui a une région présentant plus de 60 % d'homologie structurale d'aminoacides avec une portion d'au moins 20 aminoacides de la séquence d'aminoacides de lymphotoxine 1-171 représentée sur la figure 1,
et qui est un anticorps dépourvu d'anticorps non neutralisants.

2. Anticorps suivant la revendication 1, qui est un anticorps monoclonal.

3. Anticorps suivant la revendication 1 ou la revendication 2, marqué avec une substance détectable.

4. Anticorps suivant la revendication 3, dans lequel la substance détectable est un marqueur fluorescent, chimioluminescent ou radio-isotopique.

5. Anticorps suivant l'une quelconque des revendications 1 à 4, qui est immobilisé sur une surface ou matrice.

6. Anticorps suivant la revendication 5, qui est immobilisé par adsorption ou par une liaison covalente à une surface ou matrice.

7. Procédé pour séparer une lymphotoxine d'une composition, qui comprend la mise en contact de l'anticorps suivant l'une quelconque des revendications 1 à 3 avec la composition dans des conditions permettant l'adsorption de la lymphotoxine sur cet anticorps, la séparation de la lymphotoxine adsorbée par l'anticorps de la composition, et la séparation de la lymphotoxine de l'anticorps.

8. Procédé non thérapeutique pour engendrer un anticorps contre une lymphotoxine, qui comprend l'immunisation d'un animal contre une lymphotoxine polymérisée avec du glutaraldéhyde, puis l'immunisation de ce même animal contre un complexe d'adsorption lymphotoxine-alun, dans lequel la lymphotoxine a la séquence d'aminoacides 1-171 représentée sur la figure 1, ou a une séquence d'aminoacides présentant plus de 60 % d'homologie structurale avec au moins une portion d'au moins 20 aminoacides de ladite séquence 1-171 et qui présente une activité cytotoxique préférentielle d'une, et/ou une réactivité croisée immunologique avec une, et/ou l'aptitude à entrer en compétition pour des récepteurs de surface cellulaire de lymphotoxine avec une, lymphotoxine cytotoxique.

## Revendications (Revendications pour l'(les) Etat(s) contractant(s) suivant(s): AT)

1. Procédé pour engendrer un anticorps contre une lymphotoxine, qui comprend l'immunisation d'un animal contre une lymphotoxine polymérisée avec du glutaraldéhyde, puis l'immunisation de ce même animal contre un complexe d'adsorption lymphotoxine-alun, dans lequel la lymphotoxine a la séquence d'aminoacides 1-171 représentée sur la figure 1, ou a une séquence d'aminoacides présentant plus de 60 % d'homologie structurale avec au moins une portion d'au moins 20 aminoacides de ladite séquence 1-171 et qui présente une activité cytotoxique préférentielle d'une, et/ou une réactivité croisée immunologique avec une, et/ou l'aptitude à entrer en compétition pour des récepteurs de surface cellulaire de lymphotoxine avec une, lymphotoxine cytotoxique.

2. Procédé suivant la revendication 1, dans lequel l'anticorps neutralise l'activité cytolytique
(i) d'une lymphotoxine ayant la séquence d'aminoacides 1-171 représentée sur la figure 1, ou
(ii) d'une lymphotoxine qui est apte à présenter une activité cytotoxique pour des cellules tumorales in vitro ou des cellules tumorales in vivo et qui a une région présentant une homologie structurale d'aminoacides supérieure à 60 % avec une portion d'au moins 20 aminoacides de la séquence d'aminoacides de lymphotoxine 1-171 représentée sur la figure 1,
et est dépourvu d'anticorps non neutralisants.

3. Procédé suivant la revendication 1 ou la revendication 2, dans lequel l'anticorps est un anticorps monoclonal.

4. Procédé suivant l'une quelconque des revendications 1 à 3, dans lequel l'anticorps est marqué avec une substance détectable.

5. Procédé suivant la revendication 4, dans lequel la substance détectable est un marqueur fluorescent, chimioluminescent ou radio-isotopique.

6. Procédé suivant l'une quelconque des revendications 1 à 5, dans lequel l'anticorps est immobilisé sur une surface ou matrice.

7. Procédé suivant la revendication 6, dans lequel l'anticorps est immobilisé par adsorption ou par une liaison covalente à une surface ou matrice.

8. Procédé pour séparer une lymphotoxine d'une composition, qui comprend la mise en contact d'un anticorps pouvant être obtenu par le procédé suivant l'une quelconque des revendications 1 à 3 avec la composition dans des conditions permettant d'adsorber la lymphotoxine sur cet anticorps, la séparation de la lymphotoxine adsorbée par l'anticorps de la composition, et la séparation de la lymphotoxine de l'anticorps.
